Europäisches Patentamt
European Patent Office
Office européen des brevets

(19)

(11) Publication number: 0 210 839 A1

(12) EUROPEAN PATENT APPLICATION

(21) Application number: 86305667.7

(22) Date of filing: 23.07.86

(51) Int. Cl.⁴: G01N 33/68 , G01N 33/531 , G01N 33/535

(30) Priority: 31.07.85 JP 170521/85

(43) Date of publication of application: 04.02.87 Bulletin 87/06

(84) Designated Contracting States: AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Moriya, Norihiko
3-2, Aza-mamegatani Yuda Inagawa-cho
Kawabe-gun Hyogo 666-02(JP)
Inventor: Kato, Koichi
2-49, Fushimidai 1-chome Inagawa-cho
Kawabe-gun Hyogo 666-02(JP)
Inventor: Nishimura, Osamu
54-16, Daiwanishi 1-chome
Kawanishi Hyogo 666-01(JP)

(74) Representative: Laredo, Jack Joseph et al
Elkington and Fife High Holborn House 52/54
High Holborn
London, WC1V 6SH(GB)

(54) **Assay method and reagents for human interleukin-2.**

(57) A sandwich method of enzyme immunoassay for measurement of human interleukin-2 gives remarkably higher sensitivity than the bioassay method. In said sandwich method, the antibodies employed are anti-recombinant human interleukin-2 antibodies of warm-blooded animal origin or their fragments.

EP 0 210 839 A1

## ASSAY METHOD AND REAGENTS FOR HUMAN INTERLEUKIN-2

The present invention relates to an assay method and reagents for human interleukin-2.

Interleukin-2 (hereinafter abbreviated to IL-2) is a lymphokine produced by T-cells stimulated by lectin or antigens. Some reports show that IL-2 possesses such bioactivities as cytotoxic T-cell induction, splenic B-cell antibody production induction and natural killer cell activation, as well as in vitro long-term T-cell growth promotion. These bioactivities have made IL-2 the focus of attention for clinical application in the treatment of immunodeficiency disease and cancer. However, investigation of its clinical effects at high doses has been slow because of its extremely small production via the cultured cell method. Rapid progress in recombinant DNA technology, however, has recently enabled large-scale production of recombinant IL-2 (hereinafte abbreviated to rIL-2), thus permitting investigation of its effects to be initiated.

The bioactivity of IL-2 has traditionally been measured using a bioassay method [Biochemical Biophysical Research Communications, 109, 363 - (1982)], in which the detection limit for blood IL-2 concentration is 1 to 10 µg/mℓ . However, as IL-2 exhibits bioactivities in a very small amount, a method of measuring it at an accuracy higher than that of the bioassay method, is urgently desired.

The inventors worked for the developement of a high sensitivity/accuracy measuring method for human IL-2. It was found that this can be achieved by using an enzyme immunoassay (EIA) method, which uses anti-IL-2 antibodies obtained by immunizing an animal with a recombinant human IL-2 and that the sensitivity of the present method is remarkably higher than that of the bioassay method. this discovery was followed by further research, leading to the present invention.

The present invention is directed to

(1) an assay method for measuring human interleukin-2, which comprises subjecting a sample to a sandwich method of enzyme-immunoassay involving the use of an antibody supported on a carrier, an antigen and an antibody labeled with a labeling agent, wherein the antibody supported on a carrier and the antibody labeled with the labeling agent are anti-recombinant human interleukin-2 antibodies of warm-blooded animal origin or their fragments,

(2) An assay kit for immunochemical assay of human interleukin-2 by the sandwich method, which comprises:

(a) an anti-recombinant human interleukin-2 antibody of warm-blooded animal origin or its fragment, and

(b) a conjugate of an anti-recombinant human interleukin-2 antibody of warm-blboded animal origin or its fragment and a labeling agent,

(3) an anti-recombinant human interleukin-2 antibody of warm-blooded animal origin or its fragement, and

(4) a conjugate of an anti-recombinant human interleukin-2 antibody of ward-blooded animal origin or its fragement and a labeling agent.

The anti-recombinant human interleukin-2 antibodies of warm-blooded animal origin employed in the present sandwich method are produced by immunizing a warm-blooded animal with a recombinant human interleukin-2 as an antigen.

In the present specificiation, the antibody which is to be supported on a carrier is sometimes referred to as first antibody, and the antibody which is to be labeled with a labeling agent is sometimes referred to as second antibody.

As the recombinant human interleukin-2 employed as an antigen, there are mentioned peptides which are produced by gene engineering techniques possessing biological or immunological activity similar to that of natural interleukin-2, or possessing IL-2-receptor-binding ability or anti-IL-2-antibody-binding ability.

As the recombinant human IL-2, there are mentioned the following:

Polypeptide (I) [Example 1 in the specification of PCT/JP84/00460 (filing date: September 26, 1984) which corresponds to EPC Patent Publication No. 176299 published on April 2, 1986] which is produced using gene engineering techniques and which has the amino acid sequence shown in Fig. 1, and its fragments having a local amino acid sequence essential to its biological or immunological activity, are applicable.

As the recombinant human IL-2, there is included a fragment lacking one amino acid from Polypeptide (I) at the amino terminus (EPC Patent Publication No. 91539), a fragment lacking four amino acids from Polypeptide (I) at the amino terminus (Japanese Patent Laid-open No. 126088/1985), and fragments lacking several amino acids from Polypeptide (I) at the carboxy terminus.

Furthermore, as the recombinant human IL-2, there are mentioned polypeptides produced by the elimination or substitution of other amino acides, as in the case of some constitutional amino acids in above-mentioned Polypeptide (I), e.g. a polypeptide produced by replacing the cysteine residue at the 125th position with a serine residue in Polypeptide (I) (Japanese Patent Laid-open No. 93093/1984 which corresponds to U.S. Patent No. 4,518,584).

Said polypeptides may have a methionine residue at the amino terminus, and a mixture of said polypeptide and the polypeptide having a methionine residue at the amino terminus may be employed.

As a warm-blooded animal, there is recommended one other than human, and there are mentioned mammals such as rabbit, sheep, goat, rat, mouse, guinea pig, cattle, horse and pig, and birds such as chicken, pigeon, duck, goose and quail.

The amount of the recombinant human IL-2 used as an antigen is chosen in the effective range fro antibody production by said animals. It is recommended that 0.1 to 10 mg of the recombinant human IL-2, e.g. 1 mg for a rabbit and 4 mg for a goat, should be administered in combination with Freund's complete adjuvant via intracutaneous injection at the back or sole of the hind foot, intramuscular injection, etc. at a frequency of 1 to 10 times at 2-week intervals. The resulting anti-human IL-2 antibodies are obtained from the serum separated by centrifuging blood from the treated animal. (In the case of a rabbit, blood is usually collected via the auricular vein 7 to 12 days after final inoculation.) For example, the serum is first subjected to salting-out using ammonium sulfate. The resulting precipitates are collected via centrifugation and dissolved in boric acid buffer solution, etc. The resulting solution is then subjected to affinity chromatography using a column with rIL-2 immobilized to a carrier [Specification of Japanese Patent Application No. 176306/1984 (filing date: August 23, 1984), which corresponds to Japanese Patent Laid-open No. 53300/1986 published on March 17, 1986], yielding purified antibody.

Via the above procedures, both first and second antibodies cab be obtained. Two different animal species may preferably by immunized for the production of first or second antibodies. The goat, etc., which can produce a relatively large amount of antibodies, is recommended for the production of first antibodies, while the rabbit, etc. is recommended for the production of second antibodies.

Thus obtained antibodies have the properties of antibody titer of $10^4$ to $10^6$ or more (EIA method). Thus obtained antibodies also recognize natural type human interleukin-2.

It is preferable that the second antibody, used as a labeled antibody, be first subjected to the following process: The antibody is first locally digested with pepsin via a conventional method, etc. to remove its C-region, yielding the antibody fragment $F(ab')_2$, which is then converted to the antibody fragment Fab' via reduction.

As the fragment of the antibody, there are mentioned $F(ab')_2$ and Fab'. The fragment $F(ab')_2$ is produced by subjecting the antibody to a conventional pepsin degradation, and the fragment Fab' is produced by reducing the fragment $F(ab')_2$ in a conventional manner. The pepsin degradation method and the reducing method are carried out in accordance with the method described, for example, in Journal of Applied Biochemistry, 4, 41-57 - (1982).

A conjugate of an antibody and labeling agent employed in the present sandwich method is produced by reacting the antibody with a labeling agent in a buffer at a pH of about 6 to 8, at a temperature of about 10°C to 50°C for about 10 minutes to 24 hours.

As the labeling agent, there may be mentioned a labeling enzyme. Examples of such labeling enzyme are peroxidase [e.g. horseradish peroxidase - (hereinafter abbreviated to HRP)], malate dehydrogenase and β-galactosidase. Among said labeling enzymes, horseradish peroxidase is preferred.

The ensyme may be formed as a maleimidated one so as to carry out the smooth reaction [Japanese Patent Laid-open No. 90054/1984 which corresponds to EPC Patent Publication No. 1098078].

The reaction to produce the conjugate is carried out, for example, by the following manner. A first, the labeling agent is reacted with the compound represented by the formula:

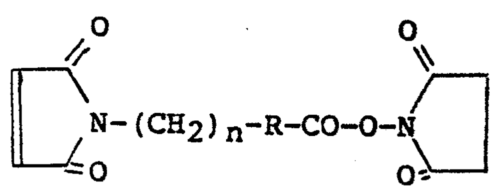

wherein n is an integer of 0 to 5 and R is a chemical bond or a divalent 6-membered cyclic hydrocarbon residue (e.g. 1,2-, 1,3-and 1,4-cyclohexylene, 1,2-, 1,3-and 1,4-phenylene),

in a buffer having a pH of about 6 to 8 at a temperature of about 10°C to 50°C for about 10 minutes to 24 hours. Said buffer is, for example, 0.1 M phosphate buffer (pH 7.0) or 0.05 M phospate buffer (pH 6.3).

The thus-obtained maleimidated labeling agent can be purified by gel chromatography, for instance. Packing materials adequate for said gel chromatography are Sephadex G-25 (Pharmacia Fine Chemical, Sweden), or Biogel P-2 (Bio-Rad Laboratories, U.S.A.), among others.

An exemplary method for reacting the maleimidated labeling agent with the second antibody is described below. The second antibody or its fragment is reduced in the presence of a mercaptoethylamine and the unreacted material is removed by gel filtration. The resultant antibody or its fragment is reacted with the maleimidated labeling agent.

Said reaction can be effected by contacting both with each other in a buffer at a temperature of about 0°C to 40°C for about 1 to 48 hours. Said buffer is, for example, 0.1 M phosphate buffer containing 5 mM sodium ethylenediaminetetraacetate (pH 6.0).

The thus-produced labeled antibody can be purified by gel chromatography, for instance. As the packing material for use in said gel chromatography, there may be mentioned, among others, Ultrogel AcA 44 (LKB, Sweden) or Sephacryl S-200 (Pharmacia Fine Chemical, Sweden).

Especially, when peroxidase is employed as the labeling enzyme, the reaction is made in the following way:

Peroxidase (horseradish peroxidase, etc.) is added to the antibody at a ratio of about 2 to 20 to 1 by weight and the mixture is reacted in a buffer solution at approx. 0 to 40°C for about 1 to 48 hours. Usable buffer solutions include a 0.1M phosphate buffer solution (pH 6.0) containing 5mM sodium ethylenediaminetetraacetate. The resulting peroxidase-labeled antibody, after purification via gel filtration etc., is used as the second antibody.

The present method of measuring human IL-2 is based on the principle and procedures of the conventional sandwich method of enzyme-immunoassay.

As the sandwich method, the procedures are, for example, disclosed in Methods in Enzymology, 84, 26 (1982); Journal of Immunological Methods 8, 223 (1975), EPC Patent Publications No. 49898, No. 88368 and No. 109078.

Examples of the sandwich method according to the present invention are as follows:

First antibody is first adsorbed to an immunoplate and treated with bovine serum albumin. A specimen solution (e.g. blood, serum, blood plasma, urine, pleural effusion, marrow fluid, extracts from various organs, etc.) is added and a conjugate of the second antibody and labeling enzyme is then added to cause a reaction. A substrate (e.g. o-phenylenediamine containing hydrogen peroxide in water, etc.) for the labeling enzyme is then added to the reaction product and absorbance of the resulting substance is measured. The IL-2 content of the specimen solution is then calculated by comparing determined absorbance to the standard curve prepared beforehand (Fig. 2). The minimum IL-2 detection limit of the present method is 10 to 40pg/mℓ.

The present method of measuring human IL-2 is described in more detail below using an example of the measurment of human IL-2 concentration in venous blood of cynomolgus monkey.

rIL-2 is intravenously administered to each cynomolgus monkey (female, 2.5 to 4 kg, 3 monkeys per group) to a dose of 0.2 to 100 µg/0.5 mℓ/kg. Blood is collected in amounts of 1 to 5 mℓ at 5, 15, 30 minutes, 1, 2, 4, 6 and 24 hours after injection. Each blood sample, after being left at room temperature for 3 to 5 hours, is centrifuged at 3,000 rpm for 10 minutes to separate serum, each of which is then diluted with a calf serum solution to 1/5, 1/10, 1/50, 1/100, 1/300 and 1/1000 concentrations. Each dilution is subjected to IL-2 measurement via the following procedure: 100 µℓ of an anti-rIL-2 goat IgG solution (protein content: 15 µg/mℓ is first placed into the well of an immunoplate (Nunc Inc.) and left at 4°C for 16 to 20 hours. The well, after removing the solution, is then washed three times with 300 µℓ of PBS. Next, 200 µℓ of 1% bovine albumin solution is added. The well, after being left at 4°C for 16 to 20 hours, is again washed with PBS. Either 100 µℓ of rIL-2 solution or a diluted specimen solution is added to the well and left at 4°C for 16 to 20 hours, after which it is washed five times with PBS. Next, 100 µℓ of anti-rIL-2 rabbit Fab'-HRP solution is added and left at room temperature for 4 hours, after which the well is again washed with PBS. Next, 100 µℓ of substrate solution is added to the well and left at room temperature in the dark for 20 to 40 minutes to cause reaction. The reaction is then stopped by adding 100 µℓ of a 2M sulfuric acid solution, after which absorbance at a wavelength of 492 nm is measured using the Titertek Multiskan MC. The rIL-2 concentration of each specimen is then calculated by comparing each determined absorbance to the standard curve for rIL-2, shown in Fig. 2. A result of the administration (rIL-2 dose: 100 µg/0.5 mℓ/kg) is shown in Fig. 4.

This result shows that the present method can exactly measure blood rIL-2 concentration with a sensitivity 100 times or more that of the bioassay method. Thus it was confirmed that the present method will provide a useful means of measuring IL-2 in clinical specimens (e.g. blood, serum, blood plasma, urine, pleural effusion, marrow fluid, etc.) and of investigating IL-2 distribution in tissues and organs.

In particular, when the anti-rIL-2 antibody, obtained by using rIL-2 as an antigen, is used, more accurate IL-2 measurement become possible due to accurate antibody properties.

An example of the assay kit of sandwich method for measuring human IL-2, and assay procedure are shown below:

1. Assay Reagents

(1) Dilution Buffer

Newborn calf serum (treated at

56°C for 30 min.) ----------------100 mℓ.

Thimerosal --------------------------0.01 g

Diluted with 0.02M phosphate buffer-saline, pH 7.0, (PBS) up to 1 liter and stored at 4°C.

(2) Bovine serum albumin (BSA) solution

Bovine serum albumin ----------------10 g

Thimerosal -------------------------0.01 g

Dissolved in 1 liter of PBS, treated at 56°C for 30 min, and stored at 4°C.

(3) Anti-rIL-2 goat IgG (first antibody)

The stock solution was diluted to 15 µg IgG/mℓ with the dilution buffer before use.

(4) Peroxidase-conjugated anti-rIL-2 rabbit Fab' (second antibody)

The stock solution was diluted to 3.5 µg protein/mℓ with the dilution buffer before use.

(5) Substrate solution

To 100 mℓ of 0.1M citrate buffer, pH 5.5, containing 0.01 % thimerosal was added 66 µℓ of a 30% $H_2O_2$ solution and the solution was stored at 4°C. The substrate solution was prepared by dissolving 44 mg of o-phenylenediamine in 10 mℓ of this solution shortly before use.

(6) rIL-2 standard solution

The stock solution of rIL-2 was diluted to a concentration of 0.04 unit/mℓ with the dilution buffer. The diluted solution was pipetted into small vials (1 mℓ each) and stored at -20°C.

2. Assay Procedure

(1) Pipette 100 µℓ of a diluted (1:25 with PBS) anti-rIL-2 goat IgG solution (15 µg/mℓ) in each well of Nunc Immunoplate and stand the plate at 4°C for 16 -24 hours.

(2) Discard the solution and wash the plate with PBS(300 µℓ in each well) 3 times.

(3) Add 200 µℓ of the BSA solution in each well and stand the plate at 4°C for 16 -24 hours.

(4) Discard the solution and wash the plate with PBS (300 µℓ) 3 times.

(5) Add 100 µℓ of test sera or a rIL-2 standard solution (0.001, 0.002, 0.004, 0.008, 0.012, 0.016 or 0.02 unit/mℓ) and stand the plate at 4°C for 16-24 hours. Use the dilution buffer to dilute test sera and rIL-2.

(6) Discard the solution and wash the plate with PBS (300 µℓ) 5 times.

(7) 100 µℓ of peroxidase-conjugated anti-rIL-2 rabbit Fab' (1:50 with the dilution buffer) and stand the plate at room temperature for 4 hours.

(8) Discard the solution and wash the plate with PBS (300 µℓ) 5 times.

(9) Add 100 µℓ of the substrate solution and stand the plate at room temperature ofr 30 min. in the dark.

(10) Add 100 µℓ of 2M $H_2SO_4$ to stop the enzyme reaction.

(11) Read the optical density at 492 nm in Titertek Multiskan.

(12) Calculate the concentration of rIL-2 in test sera from the standard calibration curve.

The present assay provides a higher sensitivity than the bioassay method. The present method is about 100 times higher than that of the bioassay method. In addition, the coefficient of correlation in measurement values is as high as 0.999 between the present method and the bioassay method; data obtained in the present method can thus be regarded as closely reflecting true specimen bioactivity (Fig. 3).

An experiment in which human IL-2 recovery was observed after addition to serum was conducted to investigate the effect of serum content in the specimen solution in the present method; it showed that when serum content is 40% or less, therefore no adverse effect on measurement data is produced.

The present assay can also measure natural type human interleukin-2.

With a sensitivity exceeding that of conventional methods, the present method of measuring human IL-2 can precisely recognize human IL-2 as a protein and thus perform accurate measurement. This method is therefore useful especially in the measurement of very small amounts of human IL-2 in clinical specimens, etc.

Brief Description of the Drawings

Fig. 1 shows an example of the amino acid sequence of recombinant human IL-2.

Fig. 2 shows the results of measurement of recombinant IL-2 and natural IL-2, obtained in Example 4.

Fig. 3 shows the correlation between the present method and bioassay method, obtained in Reference Example 2.

Fig. 4 shows the results of cynomolgus monkey blood rIL-2 concentration measurement, obtained in Example 5.

Fig. 5 shows the results of measurement of recombinant IL-2, which has been subjected to inactivation, obtained in Reference Example 3.

In the Fig. 1, amino acid abbreviations are based on those recommended by the IUPAC-IUB Commission on Biochemical Nomenclature, or those used commonly in the related field, and the amino acid represent L-type.

## Examples

The present invention is described in more detail below with Reference Examples and Examples. It should be boted that the present invention is not limited to them.

The following materials were used in the following Reference Examples or Examples.

rIL-2: Produced in accordance with the method described in Example 6, Japanese Patent Laid-open No. 115528/1985 which corresponds to EPC Patent Publication No. 145390. Three protein concentrations, 0.75, 1.195 and 1.25 mg/mℓ (5 mM ammonium acetate, pH 5.0) were used.

Bovine albumin solution: Bovine albumin (Cohn Fraction V, supplied by Daiichi Kagaku, Japan) was dissolved in a 0.02M phosphate buffer solution containing 0.145M sodium chloride (pH 6.8, abbreviated to PBS hereinafter) to a concentration of 1% and then heated at 56°C for 30 minutes. Thimerosal (Sigma Inc) was then added to a concentration of 0.001% and the solution was stored at 4°C.

New born calf serum solution: Calf serum (M.A. Biproduct Inc.) was heated at 56°C for 30 minutes and immediately filtered through a paper filter - (Toyo Filter No. 5C). The resulting filtrate was dissolved in a 0.02M PBS to a concentration of 10%. Thimerosal was then added to a 0.001% concentration and the solution was stored at 4°C.

Horseradish peroxidase conjugated anti-rabbit IgG (abbreviated to anti-rabbit IgG-HRP (Miles-Yeda Inc.) was used after dilution to a 1/40000 concentration with calf serum solution.

Horseradish peroxidase conjugated anti-goat IgG (abbreviated to anti-goat IgG-HRP): Anti-goat IgG-HRP (Cappel Inc.) was used after dilution to a 1/40000 concentration with calf serum solution.

Protein content determination: Protein concentrations were determined using the Protein Assay Kit (Bio-Rad Laboratories Ltd.) with bovine serum albumin (Sigma Inc. serving as standard.

Substrate solution: 30% hydrogen peroxide in water was added to a 0.1M citrate buffer solution containing 0.01% thimerosal (pH 5.5) to a concentration of 0.066% and then stored in a refrigerator. Just before each experiment, o-phenylenediamine was dissolved in the solution to a concentration of 40mM.

<u>Reference Example 1</u> (Production of Immobilized rIL-2)

One gram of Sepharose 4B (Pharmacia AB) activated with cyanogen bromide was placed in a glass filter and fully swollen by adding 20 mℓ of 0.001N hydrochloric acid, after which it was washed well with 200 mℓ of 0.001N hydrochloric acid. Separately, 1 mℓ of 1M sodium bicarbonate solution was added to 10 mℓ of rIL-2 solution - (protein content: 1.25 mg/mℓ; the resulting solution was adjusted to pH 8.4 with a 1N sodium hydroxide solution. To the resulting solution, cyanogen bromide-ativated Sepharose 4B treated as above was added, after which the mixture was kept at 4°C for 16 hours to cause a reaction. After reaction completion, the resulting gel was placed in a glass filter and washed with 100 mℓ of 0.1M sodium bicarbonate solution. The washed gel was then added to 10 ml of sodium bicarbonate solution containing 1M ethanolamine and left at 4°C for 2 hours while stirring slowly to cause a reaction for masking remaining active groups. The gel was then washed with 100 mℓ of 0.1M sodium bicarbonate solution, 100 mℓ of 0.1M acetic acid containing 1M sodium chloride (pH 4.0) and 100 mℓ of 0.1M borate buffer solution containing 1M sodium chloride (pH 8.0), in that order. The washed gel was then packed in a column in suspension in a 0.02M borate buffer solution (pH 8.0) and stored at 4°C. The amount of rIL-2 adsorbed to the column was approx. 11 mg.

<u>Example 1</u> (Production of anti-rIL-2 goat IgG)

(a) Production of anti-rIL-2 goat serum:

Four mℓ of rIL-2 solution (protein content: 0.75 mg/mℓ) and 4 mℓ of Freund's complete adjuvant - (Difco Laboratories Inc.) were mixed together. The resulting solution was intramuscularly administered to a goat 7 times at 2-week intervals and blood was then collected from the goat. The blood was left at room temperature for 5 hours at 4°C for 16 hours

and then centrifuged at 3,000 rpm for 10 minutes, yielding a supernatant to be used as an anti-rIL-2 goat serum.

(b) Production of anti-rIL-2 goat IgG:

Twenty mℓ of the anti-rIL-2 goat serum obtained via the procedure (a) was placed in a beaker and ammonium sulfate was added to a saturation concentration of 40% while stirring, after which it was left at 4°C for 16 hours. The resulting solution was then centrifuged at 10,000 rpm for 10 minutes, yielding a supernatant and various precipitates. After removal of supernatant,the resulting precipitates were dissolved in 35 mℓ of a 0.02M borate buffer solution of pH 8.0 and then subjected to dialysis against the same buffer solution at 4°C for 16 hours using a Spectropore membrane - (Spectrum Medical Inc.), after which centrifugation was conducted at 10,000 rpm for 10 minutes to separate precipitates from supernatant. The fifty mℓ of resulting supernatant was then halved and passed through a column (1.2 × 4cm) packed with gel-immobilized rIL-2, prepared via the process shown in Reference Example 1. The column was then fully washed with a 0.02M borate buffer solution (pH 8.0), and then with a 0.1M acetate buffer solution (pH 4.5) until absorption at the 280nm wavelength disappeared. The washed column was then subjected to elution with a 0.2M glycine hydrochloride buffer solution (pH 2.0). 1M $Na_2HPO_4$ was immediately added to the resulting elution fraction in a ratio of 1/10. The resulting solution, after adjustment to pH 7.0 with a 1N sodium hydroxide solution, was subjected to dialysis against a 0.02M borate buffer solution (pH 8.0) at 4°C for 16 hours, after which it was centrifuged at 10,000 rpm for 10 minutes, yielding a supernatant to be used as an anti-rIL-2 goat IgG. The resulting supernatant was stored at 4°C with thimerosal added to a 0.001% concentration.

(c) Properties of anti-rIL-2 goat IgG:

(1) Protein content of 0.1 to 0.8 mg/mℓ

(2) An antibody titer of $10^4$ to $10^6$ or more (EIA method)

(3) Purity of 80 to 90% or more

<u>Example 2</u> (Production of anti-rIL-2 rabbit IgG)

(a) Production of anti-rIL-2 rabbit serum:

One mℓ of rIL-2 (protein content: 1.195mg/mℓ) and 1mℓ of Freund's complete adjuvant were mixed together. The resulting mix solution was intramuscularly administered to a NIBS rabbit (male, Rabbiton Stock Farm) in the back and thigh 3 times at 2-week intervals. One week after the final injection, blood was collected via the central ear vein and left at 4°C for 16 hours, after which it was centrifuged at 3,000 rpm for 10 minutes, yielding a supernant to be used as an anti-rIL-2 rabbit serum.

(b) Production of anti-rIL-2 rabbit IgG:

Anti-rIL-2 rabbit IgG was produced via the same procedures as descrived in Example 1 (b).

(c) Properties of anti-rIL-2 rabbit IgG:

(1) Protein content of 0.1 to 0.8mg/mℓ

(2) An antibody titer of $10^4$ to $10^6$ or more (EIA method)

(3) Purity of 80 to 90% or more

<u>Example 3</u> (Production of conjugate of an antibody and peroxidase)

(a) Production of anti-rIL-2 rabbit fragment Fab' :

Using a collodion bag (Sartorius Inc.), 28mℓ of the anti-rIL-2 rabbit IgG obtained in Example 2 (b) was concentrated to approx. 1.5mℓ. The resulting concentrate was subjected to dialysis against a 0.1M acetate buffer solution (pH 4.5) at 4°C for 16 hours, after which it was centrifuged at 10,000 rpm for 10 minutes to separate precipitates from supernatant. The supernatant, after addition of 120µg of pepsin (Sigma Inc., 4,500 unit/mg), was kept at 37°C for 16 hours to cause a reaction. The reaction liquid, after addition of 299µℓ of 0.2M $Na_2HPO_4$, was adjusted to pH 8.0 with a 1N sodium hydroxide solution, after which it was subjected to gel filtration using Sephadex G-150 (column size: 1.5 × 45cm), yielding a solution containing the antibody fragment $F(ab')_2$. The resulting solution was then concentrated to approx. 1.5mℓ at 4°C using a collodion bag. The concentrate was subjectd to dialysis against a 0.1M acetate buffer solution (pH 5.0) at 4°C for 2 hours. The resulting dialyzate liquid, after addition of 0.4M of 2-mercaptoethylamine to an final concentration of 20mM, was kept at 37°C for 90 minutes to cause a reaction. The reaction liquid was then passed through a column (1.0 × 80cm) packed with Sephadex G-25 (Pharmacia AB) and fractionation was then conducted using a 0.1M phosphate buffer solution containing 5mM sodium ethylenediaminetetraacetate (pH 6.0) as a developer. This yielded anti-rIL-2 rabbit Fab' in the form of a fraction whose absorption wavelength is 280nm, which was stored at 4°C.

(b) Production of maleimidated horseradish peroxidase:

Ten mg of horseradish peroxidase (abbreviated to HRP, avialable from Boehringer Mannhein AG) was dissolved in 1.4mℓ of 0.1M phosphate buffer solution (pH 6.8). The resulting solution, after addition

of 100µℓ of N-dimethyl-formamide solution containing 4.18mg of N-Hydroxysuccinimide N-cyclohexylmethylmaleimidocarboxylate, was left at room temperature for 1 hour to cause a reaction. The reaction liquid was then centrifuged at 2,500 rpm for 10 minutes, yielding a supernatant. the supernatant was placed in a column (1.0 × 80cm) packed with Sephadex G-25 and subjected to elution using 0.1M phosphate buffer solution (pH 6.8) as a developer, yielding maleimidated horseradish peroxidase as the main fraction.

(c) Production of horseradish peroxidase conjugated anti-rIL-2 rabbit Fab' (anti-rIL-2 rabbit Fab'-HRP):

The anti-rIL-2 rabbit Fab' and maleimidated horseradish peroxidase obtained in (a) and (b), respectively, were mixed together and then concentrated to approx. 1mℓ at 4°C using a collodion bag. The resulting concentrate was then left at 4°C for 20 hours to cause a reaction. The reaction liquid was placed in a column (1.0 × 80cm) packed with Ultrogel AcA44 (LKB Inc.) and subjected to elution using 0.1M phosphate buffer solution as a developer, yielding an active fraction which, after additon of both 0.1% bovine albumin and 0.001% thimerosal, was stored at 4°C.

Example 4 (Measurement of serum rIL-2 concentrations)

First, 100µℓ of the ati-rIL-2 goat IgG solution - (protein content: 15µg/mℓ) obtained in Example 1 - (b) was placed in the well of an immunoplate (Nunc Inc.) and then left at 4°C for 16 to 20 hours. The well, after removing the solution, was washed 3 times eith 300µℓ of PBS. Next, 200µℓ of a 1% bovine albumin solution was added , after which the well was left at 4°C for 16 to 20 hours and again washed with PBS. Either 100µℓ of rIL-2 solution or a specimen solution diluted with a calf serum solution was added to the well and left at 4°C for 16 to 20 hours, after which the well was washed with PBS. Next, 100µℓ of the anti-rIL-2 rabbit Fab'-HRP solution obtained in Example 3 (c) was added and left at room temperature for 4 hours, after which the well was again washed with PBS. Next, 100µℓ of the substrate solution was added to the well and left at room temperature in the dark for 20 to 40 minutes to cause a reaction. After terminating the reaction by adding 100µℓ of 2M sulfuric acid solution, the absorbancy of each specimen solution at a wavelength of 492nm was measured using the Titertek Multiskan MC (Flow Laboratories Inc.). The results obtained are shown in Fig. 2 as -O -.

The measurement of natural IL-2 concentrations was carried out in the manner as above. The natural IL-2 employed was produced by the manner described in Biochemical and Biophysical Research Communications, Vol. 127, No. 1. pages 180-190 (1985).

The results of the measurement of natural IL-2 are shown in Fig. 2 -●-.

Example 5 (Measurement of blood rIL-2 concentrations in cynomolgus monkeys receiving rIL-2 via intravenous injection)

rIL-2 was intravenously administered to cynomolgus monkeys (female, weighting 2.5 to 4kg, three monkeys per group) at a dose of 100µg/0.5mℓ/kg. Three mℓ of blood was collected from each monkey at 5, 15, 30 minutes, 2, 2, 4, 6 and 24 hours after injection. Each blood sample was left at room temperature for 3 hours and then centrifuged at 3,000 rpm for 10 minutes to separate sample serum. The measurement of rIL-2 was carried out in the manner of Example 4. The results are shown in Fig. 4.

Reference Example 2 (Correlation in measurement data between the present method and the bioassay method)

rIL-2 was added to RPMI-1640 media containing 20% bovine fetal serum to concentrations of 3.75, 7.5, 15, 30 and 60ng/mℓ. The rIL-2 content of each medium was then measured by the manner of Exmaple 4 and the bioassay method described in Biochemical and Biophysical Research Communications vol. 109, No. 2, pp. 363-369 (1982).The results are shown in Fig. 3.

The coefficient of correlation in measurement results (r) was 0.999 between the two methods in 5 repeated experimental trails (n=5). The correlation was expressed by the linear equation $Y = 1.02X + 1.04$.

Reference Example 3

Samples containing 145 to 170 µg/ml of rIL-2 were treated with pH12 at 37°C for 5, 15, 30 min and 1, 2, 3 hours, or the same samples were treated at 75°C for 0.5, 1, 2 and 5 hours.

Thus obtained products were subjected to the measurements of the present sandwich method of the manner of Example 4, and bioassay method of the manner of Reference Example 2.

The results are shown in Fig. 5. The coefficient of correlation in measurement results(r) was 0.989 between the two methods in 22 repeated experimental trials (n = 22). The correlation was expressed by the linear equation Y = 1.06X + 3.74.

## Claims

1. An assay method for measuring human interleukin-2, which comprises subjecting a sample to a sandwich method of enzyme-immunoassay involving the use of an antibody supported on a carrier , an antigen and an antibody labeled with a labeling agent, wherein the antibody supported on a carrier and the antibody labeled with the labeling agent are anti-recombinant human interleukin-2 antibodies of warm-blooded animal origin or their fragments.

2. An assay method as claimed in Claim 1, wherein the recombinant human interleukin-2 is a polypeptide whose amino acid sequence is shown in Fig. 1 as follows:

```
1
Ala Pro Thr Ser Ser Ser Thr Lys Lys Thr Gln Leu Gln
                        20
Leu Glu His Leu Leu Leu Asp Leu Gln Met Ile Leu Asn

Gly Ile Asn Asn Tyr Lys Asn Pro Lys Leu Thr Arg Met
40
Leu Thr Phe Lys Phe Tyr Met Pro Lys Lys Ala Thr Glu
                            60
Leu Lys His Leu Gln Cys Leu Glu Glu Glu Leu Lys Pro

Leu Glu Glu Val Leu Asn Leu Ala Gln Ser Lys Asn Phe
    80
His Leu Arg Pro Arg Asp Leu Ile Ser Asn Ile Asn Val
                                100
Ile Val Leu Glu Leu Lys Gly Ser Glu Thr Thr Phe Met

Cys Glu Tyr Ala Asp Glu Thr Ala Thr Ile Val Glu Phe
        120
Leu Asn Arg Trp Ile Thr Phe Cys Gln Ser Ile Ile Ser
        133
Thr Leu Thr .           Fig. 1
```

3. An assay kit for immunochemical assay of human interleukin-2 by the sandwich method, which comprises:

(a) an inti-recobinant human interleukin-2 antibody of warm-blooded animal origin or its fragement, and

(b) a conjugate of an anti-recombinant human interleukin-2 antibody of warm-blooded animal origin or its fragment and a labeling agent.

4. An assay kit as claimed in Claim 3, wherein the recombinant human interleukin-2 is a polypeptide whose amino acid sequence is shown in Fig. 1 in Claim 2.

5. An assay kit as claimed in Claim 3, wherein the fragment is an antibody fragment Fab'.

6. An assay kit as claimed in Claim 3, wherein the antibody is purified by affinity chromatography.

7. An assay kit as claimed in Claim 3, wherein the labeling agent is horseradish peroxidase.

8. an anti-recombinant human interleukin-2 antibody of warm-blooded animal origin or its fragment.

9. An antibody as claimed in Claim 8, wherein the recombinant human interleukin-2 is a polypeptide whose amino acid sequence is shown in Fig. 1 in Claim 2.

10. A conjugate of an anti-recombinant human interleukin-2 antibody of warm-blooded animal origin or its fragement and a labeling agent.

11. A conjugate as claimed in Claim 10, wherein the recombinant human interleukin-2 is a polypeptide whose amino acid sequence is shown in Fig. 1 in Claim 2.

1
Ala Pro Thr Ser Ser Ser Thr Lys Lys Thr Gln Leu Gln

20
Leu Glu His Leu Leu Leu Asp Leu Gln Met Ile Leu Asn

Gly Ile Asn Asn Tyr Lys Asn Pro Lys Leu Thr Arg Met

40
Leu Thr Phe Lys Phe Tyr Met Pro Lys Lys Ala Thr Glu

60
Leu Lys His Leu Gln Cys Leu Glu Glu Glu Leu Lys Pro

Leu Glu Glu Val Leu Asn Leu Ala Gln Ser Lys Asn Phe

80
His Leu Arg Pro Arg Asp Leu Ile Ser Asn Ile Asn Val

100
Ile Val Leu Glu Leu Lys Gly Ser Glu Thr Thr Phe Met

Cys Glu Tyr Ala Asp Glu Thr Ala Thr Ile Val Glu Phe

120
Leu Asn Arg Trp Ile Thr Phe Cys Gln Ser Ile Ile Ser

133
Thr Leu Thr

Fig.1

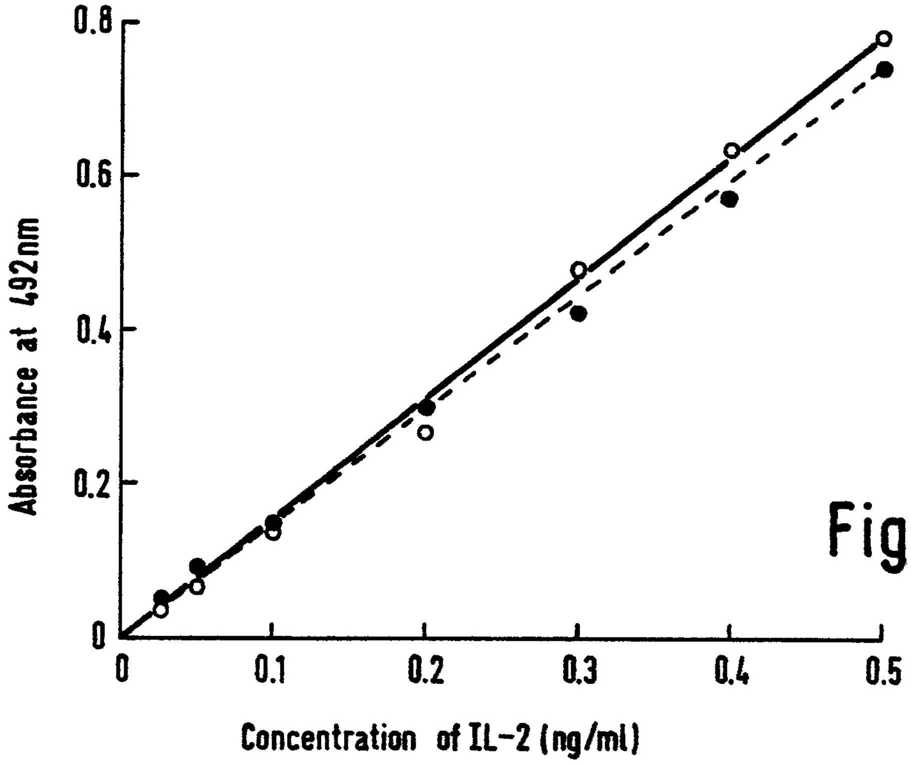
0.8
0.6
0.4
0.2
0
Absorbance at 492nm
0
0.1
0.2
0.3
0.4
0.5
Concentration of IL-2 (ng/ml)

Fig.2

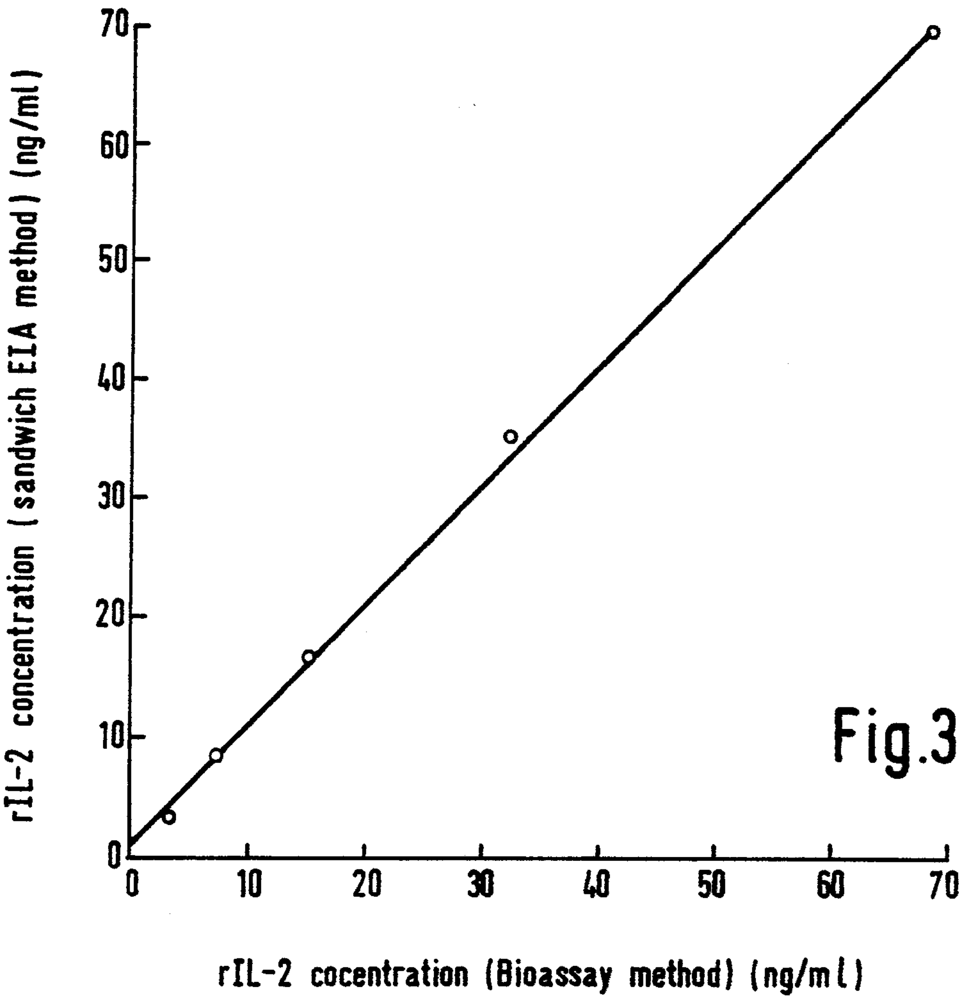
70
60
50
40
30
20
10
0
rIL-2 concentration ( sandwich EIA method) (ng/ml)
0
10
20
30
40
50
60
70
rIL-2 cocentration (Bioassay method) (ng/m l)

Fig.3

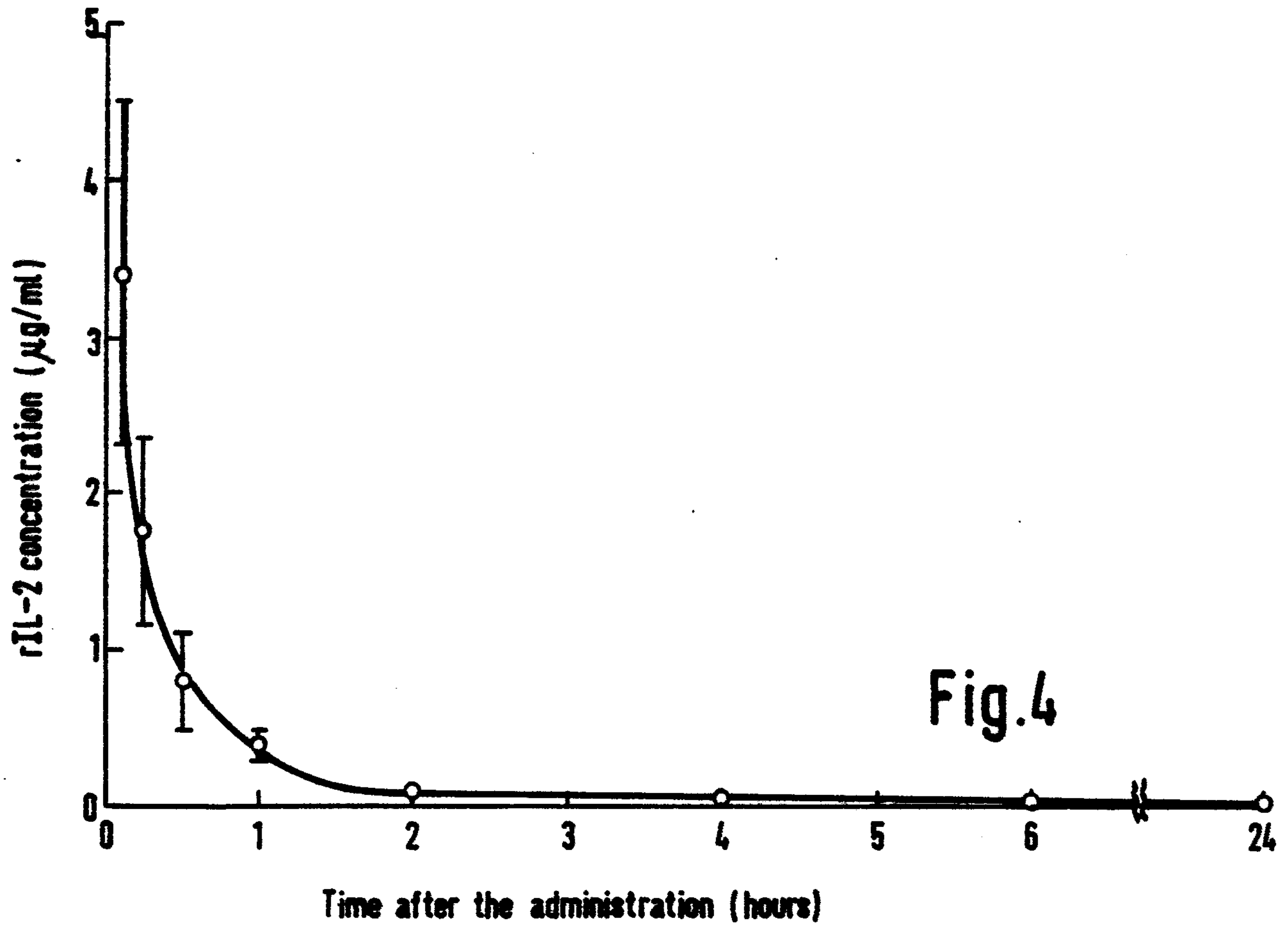
rIL-2 concentration (µg/ml)
5
4
3
2
1
0
0
1
2
3
4
5
6
24
Time after the administration (hours)

Fig.4

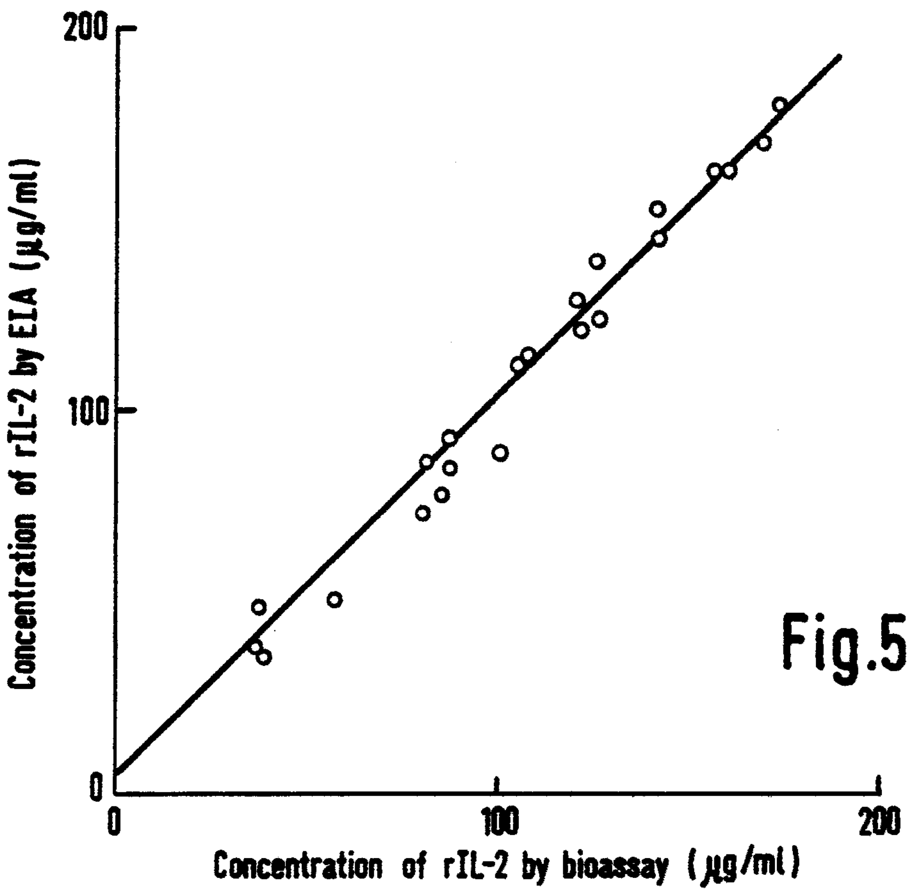
Concentration of rIL-2 by EIA (µg/ml)
200
100
0
0
100
200
Concentration of rIL-2 by bioassay (µg/ml)

Fig.5

European Patent Office

# EUROPEAN SEARCH REPORT

Application number

EP 86 30 5667

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PROCEEDINGS OF THE NATIONAL ACADEMY OF SCIENCES OF THE USA, vol. 81, April 1984, Pages 2176-2180, Washington, US; A. ALTMAN et al.: "Antibodies of predetermined specificity against chemically synthesized peptides of human interleukin 2" * Page 2176; page 2177, column 1, lines 1-13,46-67; page 2177, column 2, lines 1-10 * --- | 1-11 | G 01 N 33/68<br>G 01 N 33/531<br>G 01 N 33/535 |
| P,Y | EP-A-0 157 643 (SCRIPPS CLINIC AND RESEARCH FOUNDATION) * Page 29, line 6 - page 34 * --- | 1-11 | |
| D,P X | EP-A-0 176 299 (TAKEDA CHEMICAL INDUSTRIES) * Figure 1; claims 1,20,21,31 * --- | 1-11 | TECHNICAL FIELDS SEARCHED (Int. Cl.4) |
| X | EP-A-0 145 390 (TAKEDA CHEMICAL INDUSTRIES) * Claims 1-4 * --- | 1,2 | G 01 N<br>C 12 N<br>C 12 P |
| D,A | EP-A-0 109 078 (TAKEDA CHEMICAL INDUSTRIES) * Claims * ----- | 1 | |

The present search report has been drawn up for all claims

| Place of search | Date of completion of the search | Examiner |
|---|---|---|
| THE HAGUE | 05-11-1986 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document